(19) **Europäisches Patentamt**

**European Patent Office**

**Office européen des brevets**

(11) **EP 1 012 230 B1**

(12) **EUROPEAN PATENT SPECIFICATION**

(45) Date of publication and mention
of the grant of the patent:
**11.01.2006 Bulletin 2006/02**

(51) Int Cl.:
*C12N 1/20* *(2006.01)*    *C12N 15/31* *(2006.01)*
*A61K 39/02* *(2006.01)*    *C12N 1/20* *(2006.01)*
*C12R 1/06* *(2006.01)*

(21) Application number: **98901408.9**

(22) Date of filing: **28.01.1998**

(86) International application number:
**PCT/GB1998/000256**

(87) International publication number:
**WO 1998/033884 (06.08.1998 Gazette 1998/31)**

(54) **RENIBACTERIUM SALMONINARUM VACCINE**

IMPFSTOFF GEGEN RENIBACTERIUM SALMONINARUM

VACCIN CONTRE RENIBACTERIUM SALMONINARUM

(84) Designated Contracting States:
**AT BE CH DE DK ES FI FR GB GR IE IT LI LU MC
NL PT SE**

(30) Priority: **30.01.1997 GB 9701897**

(43) Date of publication of application:
**28.06.2000 Bulletin 2000/26**

(73) Proprietors:
• **Novartis AG**
**4056 Basel (CH)**
Designated Contracting States:
**BE CH DE DK ES FI FR GB GR IE IT LI LU MC NL
PT SE**
• **Novartis Pharma GmbH**
**1230 Wien (AT)**
Designated Contracting States:
**AT**

(72) Inventors:
• **GRIFFITHS, Steven, Gareth**
**Fredericton, New Brunswick E3B 1N9 (CA)**
• **SALONIUS, Kira**
**Cornwall, Prince Edward Island C0A 1H0 (CA)**

(56) References cited:
**WO-A-96/11707**

• **KOCH C ET AL: "16S rDNA studies on membranes
of Arthrobacter and Micrococcus: An aid for their
future taxonomic restructuring." FEMS
MICROBIOLOGY LETTERS 123 (1-2). 1994.
167-171, XP002066096**

**Description**

Protection of farmed fish against bacterial disease caused by *Renibacterium salmoniarun* by the use of a live strain of *Arthrobacter* spp. The working designation of this species, RSxII, is used through this document.

This invention relates to the protection of farmed fish against disease caused by the bacterial species *Renibacterium salmoninarum.* This disease colloquially named bacterial kidney disease or BKD from some aspects of it pathology, is one of the most economically serious diseases in salmonoid culture. Conservative estimates suggest that losses on the west coast of Canada exceed 20 million dollars annually. Similar problems have occurred in Chile and the Pacific coast of the USA. The farming of some species, such as Chinook and Coho salmon, has become economically unsustainable in these areas due to this disease. In cooler waters such as Eastern canada and Northern Europe, the disease is characterised by less severe symptoms and gives rise generally to chronic infections. The consequent poor growth performance and increased susceptibility to concurrent disease cause a high economic loss in these industries also.

A number of the standard methods for the production of effective vaccines have been used in efforts to provide protection against *Renibacterium salmoninarum.* Generally these have proved to be ineffective and, where successes have been reported by particular groups, these have provided unreplicable in the hands of others. Such methods have employed killed cells and cell fragments with or without adjuvants. For example, WO9611707 discloses a vaccine and method using killed Renibacterium salmoninarum devoid of the cell-surface associated protein, p 57.

The key factor in this lack of success is probably the ability of *Renibacterium* to survive and possibly multiply within the macrophages of the host fish. In this situation it is protected from the main immune systems of the host. Constant "leakage" of cells from the macrophages causes a low-level persistent infection which constantly challenges the fish immune system. Controlling this under normal conditions lowers the fitness of the animal and, if a further environmental or disease stress occurs, the Renibacterial cells may initiate a more damaging infection. Sometime during this process a full immune response may be mounted to the disease but this proves to be ineffective since large quantities of a 57000 kilodalton protein are produced by *Renibacterium* which induces the production of large quantities of antibodies which are not protective. The "preoccupation" of the humoral immune system with this protein prevents an effective response being made to other components of the bacteria which might confer protection. The p57 protein therefore acts as an effective decoy.

The most successful of the approaches to vaccination against *Renibacterium* have all used Freund's Complete Adjuvant (FCA). This aids in the effective presentation of antigens to the T-cells in the normal way but is also, independently, a powerful stimulator of the non-specific cellular immune responses. FCA contains cell wall fragments obtained from species of *Corynebacterium.* The taxonomic relationships between bacteria recognised under this and associated genera are not clear and Renibacteria were originally classified as Corynebacteria. Some strains of Renibacteria also have powerful stimulators of non-specific immunity on their cell surfaces further suggesting a close taxonomic relationship. The closely related genus *Arthrobacter* also contains species which have similarly reactive groups on their surface capable of stimulating non-specific immunity. Cells of this genus, not capable of causing disease but containing such groups on their surface and probably also antigens in common with *Renibacterium,* might reasonably be expected to stimulate powerful specific and non-specific immunity conferring protection against disease. The use of such *Arthrobacter* as live cells, capable of surviving inside macrophages, would prolong the stimulation and extend protection for a commercially acceptable period of time.

It is an object of the present invention to provide an improved vaccine against *Renibacterium salmonarium.*

Accordingly the present invention provides an immune stimulating agent or vaccine comprising a live, non-virulent culture of an *Arthrobacter* strain.

The invention further provides a vaccine directed to *Renibacterium salmoninarium* comprising a live non virulent culture of an *Arthrobacter* strain. The *Arthrobacter* strain is based on or is derived from strain RSxII, as deposited under Accession No ATCC 55921 with the America Type Culture Collection on 20 December 1996.

Suitably the strain is characterised by a partial 16s DNA sequence derived from the following:

GAGTTTGATCCTGGCTCAGGATGAACGCTGGCGGCGTGCTTAACACATGCAAGTC
GAACGATGAACCTGTGCTTGCACGG

GGGATTAGTGGCGAACGGGTGAGTAACACGTGAGTAACCTGCCCTTGACTTCGGG
ATAAGCCTGGGAAACTGGGTCTAAT

ACTGGATACGACCTCTCATCGCATGGTGTCCCCCTGGAAAGTTTTTGCGGTTTTG
GATGGACTCGCGGCCTATCAGCTTG

TTGGTGAGGTAATGGCTCACCAAGGCGACGACGGGTAGCCGGCCTGAGAGGGTGA
CCGGCCACACTGGGACTGAGACACG

GCCCAGACTCCTACGGGAGGCAGCAGTGGGGAATATTGCACAATGGGCGAAAGCC
TGATGCAGCGACGCCGCGTGAGGGA

CGACGGCCTTCGGGTTGTAAACCTCTTTCAGTAGGGAACAAGGCATCATTTTTGT
GGTGTTGAGGGTACTTGCAGAAGAA

GCACCGGCTAACTACGTGCCAGGCGCCGCGGTAATACGTAGGGTGCAAGCGTTAT
CCGGAATTATTGGGCGTAAAGAGCT

CGTAGGCGGTTTGTCGCGTCTTTCGTGAAAGTCCGGGGCTCAACTCCGGATCTTC
GGTGGGTACGGGCAGACTAGAGTGA

TGTAGGGGAGACTGGAATTCCTGGTGTAGCGGTGGAATGCGCAGATATCAGGAGG
AACACCGATGGCGAAGGCAGGTCTC

TGGGCATTAACTGACGCTGAGGAGCGAAAGCATGGGGAGCGAACAGGATTAGATA
CCCTGGTAGTCC

The invention further provides a pharmaceutical preparation comprising a live, non-virulent culture of an *Arthrobacter* strain.

Suitably the preparation can be used to provide protection against Renibacterium *salmonarium.*

The strain may be characterised by any or all of the following:-

1. Positive gram-stain; easily discoloured

2. Non-motile

3. The cells, in the log phase of growth, are 0.8 - 1.2 x 1.0-8.0$\mu$m often V-shaped with clubbed ends. As growth proceeds into stationary phase the rods segment into small cocci, 0.6-1.0$\mu$m in diameter.

4. The enzymatic reactions used in diagnosis are as follows where + indicates positive, - indicates negative and (+) indicates a weak positive:

| i) | Alkaline phosphatase | + |
| ii) | Butyrate esterase ($C_4$) | + |
| iii) | Caprylate esterase ($C_8$) | + |
| iv) | Myristate lipase ($C_{14}$) | - |
| v) | Leucine arylamidase | + |
| vi) | Valine arylamidase | (+) |
| vii) | Cystine arylamidase | - |
| Viii) | Trypsin | + |
| ix) | Chymostrypsin | - |
| x) | Acid Phosphatase | + |
| xi) | Phosphoamidase | - |
| xii) | $\alpha$-Galactosidase | - |

Table continued

| | | |
|---|---|---|
| xiii) | β-Galactosidase | (+) |
| xiv) | β-Glucuronidase | + |
| xv) | α-Glucosidase | + |
| xvi) | β-Glucosidase | - |
| xvii) | N-Acetyl-β-glucosamidase | - |
| xviii) | α-Mannosidase | + |
| xix) | α-Fucosidase | - |

5. Catalase Reaction Positive

6. Oxidase Reaction Negative

Suitably the immune stimulating agent/vaccine is presented as a lyophilised culture.

Preferably the vaccine comprises a lyophilised culture in combination with a sterile diluent.

The immune stimulating agent/vaccine may be administered by standard methods of vaccination.

The invention also comprises the use of an immune stimulating agent/vaccine as hereinbefore defined for the protection of salmonoid fish against *Renibacterium salmoninarum.*

The invention is an immune-stimulating agent or vaccine comprised of a live, non-virulent culture of an Arthrobacter strain RSxII as deposited under Accession No. ATTCC 55921 with the American Type Culture Collection. It would be presented as a lyophilised culture in a ready to use form in a sterile diluent to be administered by any of the standard methods used for the vaccination of fish.

Efficacy

1. The strain RSxII shares highly specific antigenic determinants with R, salmoninarum. Polyclonal antisera raised against R. Salmoninarum has a high, cross-reactive titre against whole cells of RSxII in an ELISA test system.

2. RSxII has been shown to stimulate the immune system of Atlantic salmon as demonstrated by lymphocyte proliferation assays.

3. It has been repeatedly shown that in direct challenge (in vivo) studies Atlantic salmon infected at 12-14 weeks by peritoneal injection with the pathogen were protected. The size of salmon ranged from 20-100g in different trials and protection was measured here by the extent of recovery of live bacteria from the anterior kidney, the commonest focus of infection in fish affected by this disease. Using relative percent culture activity (RPCA) as an index protection ranged from 57-87% in trials where the level of infection in non-vaccinated fish was always greater than 80%. RPCA is derived as follows:

$$\text{RPCA} = 1- \frac{[\% \text{ fish cultured positive in vaccinates}]}{[\% \text{ fish cultured positive in controls}]} \times 100$$

4. PCR was used to assess the presence of DNA of the pathogen shed by fish into the holding water as a further, very sensitive measure, of the presence of the pathogen in treated and control populations. Whereas DNA was present in the holding water of non-vaccinated fish it was present as a trace or absent from that of the vaccinates. The levels correlated well with the levels obtained by the culture technique validating that method.

The vaccine disclosed herein protects fish against *Renibacterium salmoninarum* to a greater extent than consistently achieved previously by any other formulation or method.

It is protective rather than a treatment and therefore reduces the changes of an infection becoming established, reduces or eliminates the requirement for drug therapy and promotes growth by retaining the fish at a higher level of fitness.

Unlike drug treatment it poses no risk to the environment since the invention comprises an organism isolated from the natural environment and which has been shown to be non-pathogenic for other animal species.

It can be administered concurrently with other vaccines within the standard routine of farm husbandry.

## Claims

1. An immune stimulating agent or vaccine comprising a live, non-virulent culture of an Arthrobacter strain RSxII as deposited under Accession No. ATCC 55921 with the American Type Culture Collection.

2. An agent or vaccine as claimed in claim 1 wherein the strain is **characterised by** a partial 16s DNA sequence comprising:

GAGTTTGATCCTGGCTCAGGATGAACGCTGGCGGCGTGCTTAACACATGC

AAGTCGAACGATGAACCTGTGCTTGCACGG

GGGATTAGTGGCGAACGGGTGAGTAACACGTGAGTAACCTGCCCTTGACT

TCGGGATAAGCCTGGGAAACTGGGTCTAAT

ACTGGATACGACCTCTCATCGCATGGTGTCCCCCTGGAAAGTTTTTGCGG

TTTTGGATGGACTCGCGGCCTATCAGCTTG

TTGGTGAGGTAATGGCTCACCAAGGCGACGACGGGTAGCCGGCCTGAGAG

GGTGACCGGCCACACTGGGACTGAGACACG

GCCCAGACTCCTACGGGAGGCAGCAGTGGGGAATATTGCACAATGGGCGA

AAGCCTGATGCAGCGACGCCGCGTGAGGGA

CGACGGCCTTCGGGTTGTAAACCTCTTTCAGTAGGGAACAAGGCATCATT

TTTGTGGTGTTGAGGGTACTTGCAGAAGAA

GCACCGGCTAACTACGTGCCAGGCGCCGCGGTAATACGTAGGGTGCAAGC

GTTATCCGGAATTATTGGGCGTAAAGAGCT

CGTAGGCGGTTTGTCGCGTCTTTCGTGAAAGTCCGGGGCTCAACTCCGGA

TCTTCGGTGGGTACGGGCAGACTAGAGTGA

TGTAGGGGAGACTGGAATTCCTGGTGTAGCGGTGGAATGCGCAGATATCA

GGAGGAACACCGATGGCGAAGGCAGGTCTC

TGGGCATTAACTGACGCTGAGGAGCGAAAGCATGGGGAGCGAACAGGATT

AGATACCCTGGTAGTCC

3. An agent or vaccine as claimed in claim 1 or claim 2 wherein the agent or vaccine is presented as a lyophilised culture.

4. An agent or vaccine as claimed in any one of claims 1-3 wherein the agent or vaccine comprises a lyophilised culture in combination with a sterile diluent.

5. Use of a live, non-virulent culture of an Arthrobacter strain according to any one of claims 1-4 in the preparation of a medicament.

6. Use of a live, non-virulent culture of an Arthrobacter strain in the preparation of a medicament according to claim 5 for the therapeutic application against bacterial kidney disease related to Renibacterium salmoninarum.

7. Use of a live non-virulent culture of an Arthrobacter strain in the preparation of a medicament according to claim 5 or claim 6 for use in salmonoid fish.

8. Use of a live non-virulent culture of an Arthrobacter strain in the preparation of a medicament according to any one of claims 5-7 for use in farmed salmonoid fish.

**Revendications**

1. Agent ou vaccin de stimulation immun comprenant culture vivante, non virulente d'une souche Arthrobacter RsxII tel que déposée sous le N° d'entrée ATCC 55921 auprès de l'American Type Culture Collection.

2. Agent ou vaccin selon la revendication 1, **caractérisé en ce que** la souche est **caractérisée par** une séquence ADN 16s partielle comprenant le Séq. ID No. 1

GAGTTTGATCCTGGCTCAGGATGAACGCTGGCGGCGTGCTTAACACATGC

AAGTCGAACGATGAACCTGTGCTTGCACGG

GGGATTAGTGGCGAACGGGTGAGTAACACGTGAGTAACCTGCCCTTGACT

TCGGGATAAGCCTGGGAAACTGGGTCTAAT

ACTGGATACGACCTCTCATCGCATGGTGTCCCCCTGGAAAGTTTTTGCGG

TTTTGGATGGACTCGCGGCCTATCAGCTTG

TTGGTGAGGTAATGGCTCACCAAGGCGACGACGGGTAGCCGGCCTGAGAG

GGTGACCGGCCACACTGGGACTGAGACACG

GCCCAGACTCCTACGGGAGGCAGCAGTGGGGAATATTGCACAATGGGCGA

AAGCCTGATGCAGCGACGCCGCGTGAGGGA

CGACGGCCTTCGGGTTGTAAACCTCTTTCAGTAGGGAACAAGGCATCATT

TTTGTGGTGTTGAGGGTACTTGCAGAAGAA

GCACCGGCTAACTACGTGCCAGGCGCCGCGGTAATACGTAGGGTGCAAGC

GTTATCCGGAATTATTGGGCGTAAAGAGCT

CGTAGGCGGTTTGTCGCGTCTTTCGTGAAAGTCCGGGGGCTCAACTCCGGA

TCTTCGGTGGGTACGGGCAGACTAGAGTGA

TGTAGGGGAGACTGGAATTCCTGGTGTAGCGGTGGAATGCGCAGATATCA

GGAGGAACACCGATGGCGAAGGCAGGTCTC

TGGGCATTAACTGACGCTGAGGAGCGAAAGCATGGGGAGCGAACAGGATT

AGATACCCTGGTAGTCC

**3.** Agent ou vaccin selon la revendication 1 ou 2, **caractérisé en ce que** l'agent ou le vaccin est présenté comme étant une culture lyophilisée.

**4.** Agent ou vaccin selon l'une quelconque des revendications 1 à 3, **caractérisé en ce que** l'agent ou le vaccin comprend une culture lyophilisée en combinaison avec un diluant stérile.

**5.** Utilisation d'une culture vivante non virulente d'une souche *Arthrobacter* selon l'une quelconque des revendications 1 à 4. dans la préparation d'un médicament.

**6.** Utilisation d'une culture vivante non virulente d'une souche *Arthrobacter* dans la préparation d'un médicament selon l'une quelconque des revendications 1 à 5 destiné à une application thérapeutique à l'encontre de la maladie bactérienne du rein apparentée à la *Renibacterium salmoninarum.*

**7.** Utilisation d'une culture vivante non virulente d'une souche Arthrobacter dans la préparation d'un médicament selon la revendication 5 ou 6 utilisable pour traiter les salmonidés.

8. Utilisation d'une culture vivante non virulente d'une souche Arthrobacter dans la préparation d'un médicament selon l'une quelconque des revendications 5-7 pour traiter les élevages de salmonidés.

**Patentansprüche**

1. Immunstimulierendes Agens oder Vakzin, das eine lebende, nicht-virulente Kultur von *Arthrobacter-Stamm* RSxII, wie er bei der American Type Culture Collection unter der Eingangs-Nr. ATCC 55921 hinterlegt wurde, ist.

2. Agens oder Vakzin nach Anspruch 1, wobei der Stamm durch eine 16s-DNA-Teilsequenz **gekennzeichnet** ist, die umfasst:
SEQ ID NO: 1:

GAGTTTGATCCTGGCTCAGGATGAACGCTGGCGGCGTGCTTAACACATGC

AAGTCGAACGATGAACCTGTGCTTGCACGG

GGGATTAGTGGCGAACGGGTGAGTAACACGTGAGTAACCTGCCCTTGACT

TCGGGATAAGCCTGGGAAACTGGGTCTAAT

ACTGGATACGACCTCTCATCGCATGGTGTCCCCCTGGAAAGTTTTTGCGG

TTTTGGATGGACTCGCGGCCTATCAGCTTG

TTGGTGAGGTAATGGCTCACCAAGGCGACGACGGGTAGCCGGCCTGAGAG

GGTGACCGGCCACACTGGGACTGAGACACG

GCCCAGACTCCTACGGGAGGCAGCAGTGGGGAATATTGCACAATGGGCGA

AAGCCTGATGCAGCGACGCCGCGTGAGGGA

CGACGGCCTTCGGGTTGTAAACCTCTTTCAGTAGGGAACAAGGCATCATT

TTTGTGGTGTTGAGGGTACTTGCAGAAGAA

GCACCGGCTAACTACGTGCCAGGCGCCGCGGTAATACGTAGGGTGCAAGC

GTTATCCGGAATTATTGGGCGTAAAGAGCT

CGTAGGCGGTTTGTCGCGTCTTTCGTGAAAGTCCGGGGCTCAACTCCGGA

TCTTCGGTGGGTACGGGCAGACTAGAGTGA

TGTAGGGGAGACTGGAATTCCTGGTGTAGCGGTGGAATGCGCAGATATCA

GGAGGAACACCGATGGCGAAGGCAGGTCTC

TGGGCATTAACTGACGCTGAGGAGCGAAAGCATGGGGAGCGAACAGGATT

AGATACCCTGGTAGTCC

**3.** Agens oder Vakzin nach Anspruch 1 oder 2, wobei das Agens oder das Vakzin als lyophilisierte Kultur präsentiert wird.

**4.** Agens oder Vakzin nach einem der Ansprüche 1 bis 3, wobei das Agens oder Vakzin eine lyophilisierte Kultur in Kombination mit einem sterilen Verdünnungsmittel umfasst.

**5.** Verwendung einer lebenden, nicht-virulenten Kultur eines Arthrobacter-Stamms nach einem der Ansprüche 1 bis 4 bei der Herstellung eines Medikaments.

**6.** Verwendung einer lebenden, nicht-virulenten Kultur eines Arthrobacter-Stamms bei der Herstellung eines Medikaments nach Anspruch 5 für die therapeutische Anwendung gegen bakterielle Nierenkrankheit, die mit *Renibacterium salmoninarum* in Verbindung steht.

**7.** Verwendung einer lebenden, nicht-virulenten Kultur eines Arthrobacter-Stamms bei der Herstellung eines Medikaments nach Anspruch 5 oder Anspruch 6 zur Verwendung in salmonidem Fisch.

**8.** Verwendung einer lebenden, nicht-virulenten Kultur eines Arthrobacter-Stamms bei der Herstellung eines Medikaments nach einem der Ansprüche 5 bis 7 zur Verwendung in salmonoidem Zuchtfisch.

SEQUENCE LISTING

(1) GENERAL INFORMATION:

(i) APPLICANT:

(A) NAME: AQUA HEALTH (EUROPE) LTD
(B) STREET: ENTERPRISE HOUSE, SPRINGKERSE BUSINESS PARK
(C) CITY: STIRLING
(E) COUNTRY: UNITED KINGDOM
(F) POSTAL CODE (ZIP): FK7 7UF

(ii) TITLE OF INVENTION: VACCINE

(iii) NUMBER OF SEQUENCES: 1

(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:

(A) LENGTH: 787 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 1:

```
GAGTTTGATC CTGGCTCAGG ATGAACGCTG GCGGCGTGCT TAACACATGC AAGTCGAACG        60

ATGAACCTGT GCTTGCACGG GGGATTAGTG GCGAACGGGT GAGTAACACG TGAGTAACCT       120

GCCCTTGACT TCGGGATAAG CCTGGGAAAC TGGGTCTAAT ACTGGATACG ACCTCTCATC       180

GCATGGTGTC CCCCTGGAAA GTTTTTGCGG TTTTGGATGG ACTCGCGGCC TATCAGCTTG       240

TTGGTGAGGT AATGGCTCAC CAAGGCGACG ACGGGTAGCC GGCCTGAGAG GGTGACCGGC       300

CACACTGGGA CTGAGACACG GCCCAGACTC CTACGGGAGG CAGCAGTGGG GAATATTGCA       360

CAATGGGCGA AAGCCTGATG CAGCGACGCC GCGTGAGGGA CGACGGCCTT CGGGTTGTAA       420

ACCTCTTTCA GTAGGGAACA AGGCATCATT TTTGTGGTGT TGAGGGTACT TGCAGAAGAA       480

GCACCGGCTA ACTACGTGCC AGGCGCCGCG GTAATACGTA GGGTGCAAGC GTTATCCGGA       540

ATTATTGGGC GTAAAGAGCT CGTAGGCGGT TTGTCGCGTC TTTCGTGAAA GTCCGGGGCT       600

CAACTCCGGA TCTTCGGTGG GTACGGGCAG ACTAGAGTGA TGTAGGGGAG ACTGGAATTC       660


CTGGTGTAGC GGTGGAATGC GCAGATATCA GGAGGAACAC CGATGGCGAA GGCAGGTCTC       720

TGGGCATTAA CTGACGCTGA GGAGCGAAAG CATGGGGAGC GAACAGGATT AGATACCCTG       780

GTAGTCC                                                                 787
```